# EUROPEAN PATENT APPLICATION

(11) **EP 4 755 308 A1**
(43) Date of publication of application: **10.06.2026**
(21) Application number: 24218229.3
(22) Date of filing: 09.12.2024
(51) Int. Cl.: A61B 6/03, A61B 6/58

(54) **FOCAL SPOT DEGRADATION PREDICTION**

(71) Applicant: Koninklijke Philips N.V., 5656 AE Eindhoven (NL)
(72) Inventor: SOSSIN, Artur, Eindhoven (NL); VAN BEERS, Paul, Eindhoven (NL); WIEGERT, Jens, 5656AG Eindhoven (NL)
(74) Representative: Philips Intellectual Property & Standards

(57) **Abstract**

Proposed concepts thus aim to provide schemes, solutions, concepts, designs, methods and systems pertaining to generating a model for predicting focal spot degradation. In particular, embodiments aim to provide a method for generating a model for predicting focal spot degradation by generating a first sub-model describing a relationship between medical image data and a focal spot shape, and a second sub-model describing a relationship between a focal spot shape and a focal spot degradation score. A composite model is then generated based on the first and second sub-models. The composite model, which can be a machine-learning model, can thus be used to predict the focal spot degradation score from the medical image data.

## Description

### FIELD OF THE INVENTION

This invention relates to the field of medical imaging technology, and more specifically to the field of predicting focal spot degradation.

### BACKGROUND OF THE INVENTION

Computed Tomography (CT) is a widely used medical imaging technique that provides detailed cross-sectional views of the body. It works by rotating an X-ray source and detector around the patient, capturing a series of projections from different angles. These projections are then reconstructed into a 3D image using complex mathematical algorithms.

One of the main components of a CT system is the X-ray tube, which generates the X-rays used for imaging. The X-ray tube consists of a cathode, which emits electrons, and an anode, which receives the electrons and emits X-rays. The point at which the electrons hit the anode is known as the focal spot. The size and shape of the focal spot have a direct impact on the resolution of the final image.

The CT landscape is continuously evolving towards higher resolution imaging, facilitated by advancements in detector pixels and electronics. However, the system resolution is not determined by these factors alone. Other contributors such as the mode of operation of the focal spot (dual or single), the size and shape of the focal spot, re-binning, and reconstruction-related filtering also play a role.

Over time, the focal spot can degrade due to various factors such as focal track roughening on the anode side, contamination in the vacuum, and filament wear on the cathode side. These degradations and changes in the focal spot shape and size can have a detrimental effect on the final image resolution.

Furthermore, these degradations in the focal spot can be indicative of the tube's imminent breakdown. Therefore, it is of great interest to track these changes and degradations in the focal spot over time. This can help in carrying out the appropriate actions to rectify the issue, such as adjusting protocols to extend tube lifetime and scheduling a timely replacement. This can result in less downtime for the customer and a more predictable behavior of the CT system.

### SUMMARY OF THE INVENTION

The invention is defined by the claims.

According to examples in accordance with an aspect of the invention, there is provided a method for generating a model for predicting focal spot degradation.

The method comprises: generating a first sub-model describing a representation of a relationship between medical image data and a focal spot shape; generating a second sub-model describing a representation of a relationship between a focal spot shape and a focal spot degradation score indicating a level of degradation in the focal spot's shape and/or size; and generating a composite model for predicting focal spot degradation based on the first sub-model and the second sub-model; wherein the medical image data comprises one of: CT projection data; a CT reconstructed image; X-ray projection data; and an X-ray reconstructed image.

Proposed concepts thus aim to provide schemes, solutions, concepts, designs, methods and systems pertaining to generating a model for predicting focal spot degradation. In particular, embodiments aim to provide a method for generating a model for predicting focal spot degradation by generating one sub-model capable of relating medical image data to focal spot shape and another sub-model capable of relating focal spot shape to focal spot degradation. A composite model can then be generated essentially relating medical image data to focal spot degradation.

In other words, there is proposed a method which can facilitate the tracking of focal spot degradation in Computed Tomography (CT) and/or X-ray systems (via the composite model). The focal spot, a resulting feature of the X-ray tube in a CT system, is subject to degradation over time due to various factors. This degradation can negatively impact the final image resolution and signal an imminent breakdown of the tube. The disclosed method facilitates the use of CT/X-ray image/projection data to track this degradation, providing a proactive approach to managing and maintaining CT/X-ray systems.

The benefit of the present invention therefore lies in its ability to generate a predictive model that leverages medical image data to assess the condition of the focal spot in medical imaging equipment. This method facilitates early detection of focal spot degradation, which can lead to improved maintenance schedules, reduced downtime for medical imaging devices, and therefore enhanced image quality for diagnostic purposes.

The inventors have realized that by first generating sub-models which can learn relationships from medical image data (e.g., image/projection data) to focal spot shape and then from focal spot shape to focal spot degradation, respectively, a composite model can then be generated which can learn a relationship directly from medical image data to focal spot degradation. This composite model can thus provide an efficient and/or effective way to determine the degradation of a focal spot of a medical imaging system purely from medical image data output by said medical system.

Ultimately, an improved method for generating a model for predicting focal spot degradation is provided.

In some embodiments, the composite model may be a machine-learning model, and wherein generating the composite model for predicting focal spot degradation may comprise: training the composite model, using the first and second sub-models, to predict, from medical image data, a focal spot degradation score. This may offer the advantage of utilizing advanced computational techniques to accurately predict focal spot degradation directly from medical image data. For example, this can result in more precise and reliable assessments of equipment condition, leading to more efficient use of medical imaging resources.

In some embodiments, the composite model may comprise at least one of: a convolutional neural network; and a transformer model. This may provide the benefit of leveraging state-of-the-art machine learning architectures known for their effectiveness in image analysis and pattern recognition. This can enhance the model's accuracy in predicting focal spot degradation.

In some embodiments, the first sub-model may comprise a machine-learning model and generating the first sub-model may comprise: training the first sub-model on training data comprising simulated medical image data, wherein the simulated medical image data has been generated using different modulation transfer functions, MTFs, corresponding to different focal spot shapes. The use of simulated medical image data to train the first sub-model may facilitate the creation of a robust and comprehensive training dataset. This can lead to a more accurate and generalizable model capable of detecting a wide range of focal spot degradation scenarios.

In some embodiments, each modulation transfer function may comprise a function of distance to an iso-center of the medical imaging machine. The modulation transfer function's dependency on the distance to the iso-center of the medical imaging machine may allow for a more nuanced and detailed understanding of the system's resolution characteristics. This can improve the precision of the predictive model.

In some embodiments, the distance to the iso-center may comprise one of: tangential distance; and radial distance. In this way, the predictive model may account for both tangential and radial distances to the iso-center, which can provide a more complete picture of the system's resolution behavior.

In some embodiments, the method may further comprise: generating the different MTFs using a simulated medical imaging machine with an adjustable focal spot shape. This may provide an effective way of generating a robust and comprehensive training dataset including a wide range of focal spot degradation scenarios.

In some embodiments, the second sub-model may comprise at least one of: a look-up table; a mathematical function; a regression model; a machine-learning model; and an algorithm. These may all be suitable forms for the second sub-model, varying in efficiency and complexity.

In some embodiments, the first sub-model may comprise at least one of: a machine-learning model; an algorithm; a regression model; and a mathematical function. These may all be suitable forms for the first sub-model, varying in efficiency and complexity.

In some embodiments, the method may further comprise generating a third sub-model describing a representation of a relationship between a focal spot degradation score and an X-ray tube degradation score indicating a level of degradation in the X-ray tube which generated the focal spot; and generating the composite model is further based on the third sub-model, such that the composite model is for predicting X-ray tube degradation. This may allow users to be provided with a more complete assessment of the medical imaging equipment's health, allowing a degradation of the X-ray tube to be predicted based on the focal spot degradation.

According to another aspect of the invention, there is provided a computer-implemented method for predicting focal spot degradation. The method comprising: obtaining a model for predicting focal spot degradation generated according to any of the herein-disclosed methods; and processing first medical image data from a single medical-imaging machine with the obtained model to predict a first focal spot degradation score. This may allow a user to actually use the generated composite model in order to predict a focal spot degradation of an actual medical imaging system based on medical image data output by said medical imaging system.

In some embodiments, the method may further comprise processing second medical image data with the obtained model to predict a second focal spot degradation score, wherein the second medical image data was captured at a later point in time to the first medical image data and by the same medical imaging machine; and predicting a timepoint when the medical imaging machine will reach complete focal spot degradation based on the first and second focal spot degradation scores and the times at which the first and second medical image data were captured. This may thus facilitate the prediction of a timeline for complete focal spot degradation. This predicative capability may be especially useful for planning maintenance and replacement of the X-ray tube, ensuring continuous operation of medical imaging services without unexpected interruptions.

According to another aspect of the invention, there is provided a computer program product comprising computer program code means which, when executed on a computing device having a processing system, cause the processing system to perform all of the steps of the computer-implemented method according to any herein disclosed method.

According to another aspect of the invention, there is provided a system for generating a model for predicting focal spot degradation. The system comprising a processing unit configured to: generate a first sub-model describing a representation of a relationship between medical image data and a focal spot shape; generate a second sub-model describing a representation of a relationship between a focal spot shape and a focal spot degradation score indicating a level of degradation in the focal spot's shape and/or size; and generate a composite model for predicting focal spot degradation based on the first sub-model and the second sub-model; wherein the medical image data comprises one of: CT projection data; a CT reconstructed image; X-ray projection data; and an X-ray reconstructed image.

In some embodiments, the composite model may be a machine-learning model, and wherein generating the composite model for predicting focal spot degradation may comprise training the composite model, using the first and second sub-models, to predict, from medical image data, a focal spot degradation score.

Thus, there may be proposed concepts for generating a model for predicting focal spot degradation, and this may be done based on the generation of two sub-models first, which can then be utilized to generate a composite model capable of predicting focal spot degradation.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for generating a model for predicting focal spot degradation according to a proposed embodiment;
Fig. 2 is a flow diagram of a method for generating a model for predicting focal spot degradation according to a proposed embodiment;
Fig. 3 is a simplified flow diagram of a method for predicting focal spot degradation according to a proposed embodiment;
Fig. 4 is a flow diagram of a method for predicting focal spot degradation according to a proposed embodiment;
Fig. 5 is a simplified block diagram of a system for generating a model for predicting focal spot degradation according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

Embodiments of the invention aim to provide a method for generating a model for predicting focal spot degradation. This can be achieved by generating one sub-model capable of relating medical image data to focal spot shape and another sub-model capable of relating focal spot shape to focal spot degradation. A composite model can then be generated essentially relating medical image data to focal spot degradation.

In other words, there is proposed a method which can facilitate the tracking of focal spot degradation in Computed Tomography (CT) and/or X-ray systems (via the composite model). The focal spot, a resulting feature of the X-ray tube in a CT system, is subject to degradation overtime due to various factors. This degradation can negatively impact the final image resolution and signal an imminent breakdown of the tube. The disclosed method leverages CT/X-ray image/projection data to track this degradation, providing a proactive approach to managing and maintaining CT/X-ray systems.

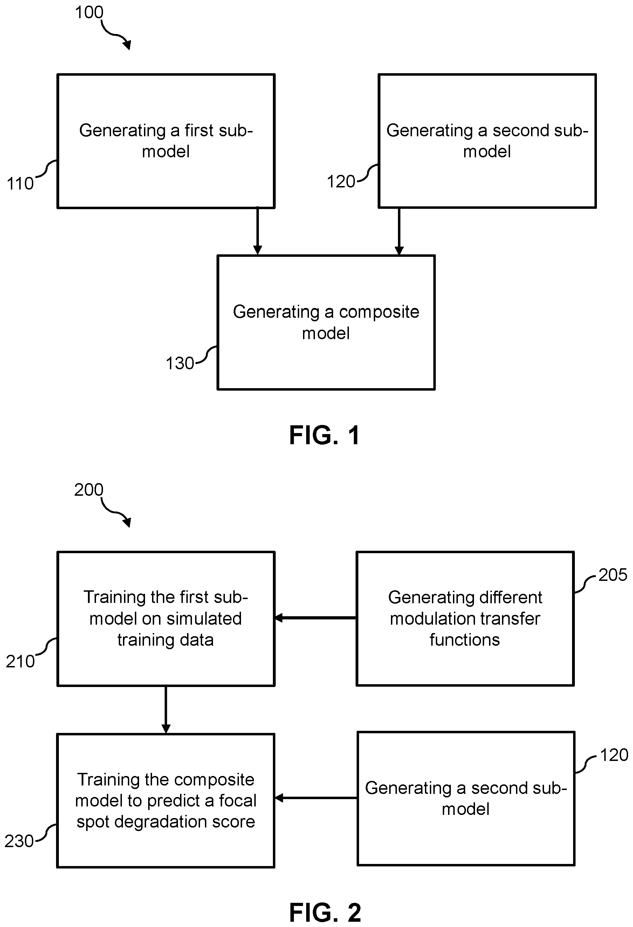

Referring now to Fig. 1, there is depicted a simplified flow diagram of a method 100 for generating a model for predicting focal spot degradation according to a proposed embodiment.

Step 110 of method 100 comprises generating a first sub-model describing a representation of a relationship between medical image data and a focal spot shape. The medical image data comprises one of: CT projection data; a CT reconstructed image; X-ray projection data; and an X-ray reconstructed image. In other words, the medical image data must be uniform in type, i.e., only CT projection data or only an X-ray reconstructed image. This allows the first sub-model to be optimized for predicting a focal spot shape based on the specific type of medical image data.

A focal spot shape refers to the geometric area on the anode of an X-ray tube where electrons collide to produce X-rays. In some respects, the shape can be round, oval, or rectangular, and its size and intensity distribution are factors that influence the resolution of the resulting X-ray/CT image. Over time, the focal spot can degrade due to factors such as thermal stress and electron bombardment, leading to changes in its shape and size, which can affect the quality of the medical images produced. In some cases, the focal spot shape is designed to optimize the balance between image resolution and X-ray intensity for specific medical imaging applications.

A sub-model can be understood as a full model (e.g., a full machine-learning model or mathematical function or look-up table, etc.) with the term 'sub' only indicating that the model is not intended to be used by an end user but instead is for the purpose of ultimately generating the composite model (in step 130). The sub-model can thus also be referred to as an intermediate model or a first-stage model or a partial model.

For example, in this embodiment, the first sub-model can comprise at least one of: a machine-learning model; an algorithm; a regression model; and a mathematical function. These are all suitable forms for the first sub-model, varying in efficiency and complexity, however, in other embodiments, as would be understood by the skilled person, any suitable form of model can be used.

Step 120 comprises generating a second sub-model describing a representation of a relationship between a focal spot shape and a focal spot degradation score indicating a level of degradation in the focal spot's shape and/or size. A level of degradation in the focal spot's shape and/or size can be understood as a deviation from the ideal or expected geometric configuration that is used to produce the sharpest possible (or the desired) image in a CT or X-ray system. Ideally, the focal spot is precisely controlled to maintain the resolution of the CT / X-ray images. Over time, however, various factors such as thermal stress, electron bombardment, and general wear and tear can cause changes in the shape and size of the focal spot.

Degradation can manifest as an increase in the size of the focal spot, which can lead to a reduction in the spatial resolution of the images. This is because a larger focal spot produces X-rays that diverge more, resulting in less sharpness and detail in the captured images. Similarly, changes in the shape of the focal spot, such as becoming more elliptical or irregular, can cause asymmetries in the X-ray beam and lead to image artifacts or blurring.

In some cases, the level of degradation (i.e., the focal spot degradation score) can be quantified by comparing the current focal spot characteristics with the original specifications or with a set of predefined standards (e.g., a predetermined size and/or shape for the focal spot). This comparison can then be quantified as the focal spot degradation score, providing an absolute or a relative deviation from the ideal focal spot characteristics (i.e., shape and/or size). For example, the focal spot degradation score can take a scalar value from 0 to 100%, or it might be categorized into discrete levels (such as nominal, degraded, strongly degraded, etc.) or it can be expressed as a percentage. Of course, the skilled person would understand that the focal spot degradation score can take any suitable form.

For example, in this embodiment, the second sub-model can comprise at least one of: a look-up table; a mathematical function; a regression model; a machine-learning model; and an algorithm. These are all suitable forms for the second sub-model, varying in efficiency and complexity, however, in other embodiments, as would be understood by the skilled person, any suitable form of model can be used.

In other words, the second sub-model can be understood as a focal spot degradation model comprising, for example, a sequence of focal spot shapes with different levels of degradation. These can be produced either via simulation or experiments with an X-ray camera. In an experimental set-up, for example, snapshots of the X-ray tube can be taken as it is put under various levels of wear and usage. In a simulation set-up, for example, field and tube knowledge can be used to model the shape change of the focal spot with continued use. Both these approaches could thus be used to create a look-up table, for example, relating focal spot shapes to a level of degradation (which can then be correlated to a certain tube usage profile).

Step 130 comprises generating a composite model for predicting focal spot degradation based on the first sub-model and the second sub-model. In other words, the first sub-model and the second sub-model are used during the generation of the composite model which is then capable of predicting focal spot degradation (e.g., based on medical image data). To be clear, the composite model thus describes a representation of a relationship between medical image data and a focal spot degradation score, thus facilitating the prediction of a focal spot degradation score based on medical image data (e.g., a CT reconstructed image) input into the composite model. As the skilled person would appreciate, the first sub-model and the second sub-model could be used in a wide variety of ways in order to help generate the composite model. For instance, in some embodiments, they can simply be combined together (e.g., end-to-end) where medical image data can be input in one end, and a focal spot degradation prediction (e.g., a focal spot degradation score) can be output the other end. In other embodiments (such as those described later), the first and second sub-models can be used during the training of an entirely new (composite) machine-learning model (e.g., to generate suitable training data for the composite model).

The composite model can also be referred to as a combined model or a joint model or a final model or an integrated model or a second-stage model or a full model.

In this embodiment, the composite model comprises a convolution neural network, but in other embodiments, the composite model can comprise a transformer model or any other suitable form of machine-learning model (e.g., a Random Forest model). This has the benefit of leveraging state-of-the-art machine-learning models known for their effectiveness in image analysis and pattern recognition. This can enhance the composite model's accuracy in predicting focal spot degradation. It should be noted that transformer models (i.e., a machine-learning model utilizing transfer-type architecture) have been seen to provide improved accuracy in multiple image restoration and computer vision tasks, albeit at the cost of heavier computational load. Therefore, depending on the deployment situation, the choice of composite model can depend on a desired balance between accuracy and computational cost.

There are several types of neural networks, such as, for example, convolutional neural networks (CNNs) and recurrent neural networks (RNNs). As stated, in embodiments of the present invention, the composite model can comprise CNN-based learning algorithms.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g., ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

It should be noted that this method 100 (and all others disclosed herein) are applicable for both conventional and spectral (photon counting) CT systems and can also be applied to other modalities where accurate modelling of both the system and the focal spot is possible, such as IGT, DXR, etc.

Referring now to Fig. 2, there is depicted a flow diagram of a method 200 for generating a model for predicting focal spot degradation according to a proposed embodiment.

Step 120 is substantially the same as has been described in relation to the method 100 in Fig. 1.

In this embodiment, the first sub-model comprises a machine-learning model and step 210 comprises training the first sub-model on training data comprising simulated medical image data, wherein the simulated medical image data has been generated using different modulation transfer functions, MTFs, corresponding to different focal spot shapes. The use of simulated medical image data to train the first sub-model facilitates the creation of a robust and comprehensive training dataset. This can lead to a more accurate and generalizable model capable of detecting a wide range of focal spot degradation scenarios.

In fact, in this embodiment, the different MTFs are actually generated in step 205 using a simulated medical imaging machine with an adjustable focal spot shape. This may provide an effective way of generating a robust and comprehensive training dataset including a wide range of focal spot degradation scenarios. In other embodiments, however, the MTFs do not need to actually be generated within the method, and instead, they can be obtained in any suitable way, e.g., experimentally or from a memory storage or provided by a user, for example.

In other words, for example, a system resolution model can be used to generate the different MTFs. For example, given the availability of sufficient system knowledge, a simulation framework can be built that enables the characterization of the medical imaging system resolution including a plurality of contributors from hardware to the reconstruction software (other contributors would be apparent to the skilled person). Of course, nominal and degraded tube-performance can be included in this model. This system characterization framework would thus be capable of producing a link between focal spot shape and spatial resolution in a CT/X-ray system. Resulting system MTFs can then be mapped out as a function of distance to the iso-center. For example, a photon counting CT system MTF dependency (at 10% level) on the distance from the iso-center can be generated. This can also be split into its two separate directions (tangential and radial).

It should be noted that a MTF can be completely radially unsymmetric depending on the focal spot operation mode (dual versus single focal spot). It should also be noted that for both modes, the resolution of the system progressively degrades with distance from the iso-center.

In this embodiment, each modulation transfer function (MTF) comprises a function of distance to an iso-center of the medical imaging machine. The modulation transfer function's dependency on the distance to the iso-center of the medical imaging machine allows for a more nuanced and detailed understanding of the system's resolution characteristics. This can improve the precision of the predictive model, but as the skilled person would understand, is not essential to the working of the invention.

Furthermore, in this embodiment, the distance to the iso-center comprises one of: tangential distance; and radial distance. In this way, the predictive model accounts for both tangential and radial distances to the iso-center, which can provide a more complete picture of the system's resolution behavior, but as the skilled person would understand, this feature is not essential to the working of the invention.

In this embodiment, the composite model is a machine-learning model and step 230 comprises training the composite model, using the first and second sub-models, to predict, from medical image data, a focal spot degradation score. This offers the advantage of utilizing advanced computational techniques to accurately predict focal spot degradation directly from medical image data. For example, this can result in more precise and reliable assessments of equipment condition, leading to more efficient use of medical imaging resources. As the skilled person would understand, the first and second sub-models could be used in a variety of suitable ways during the training of the composite model. For example, the first and second sub-models can be used to generate suitable training data for training the composite model, for example, wherein the known input is medical image data and the known output is a focal spot degradation score. For example, in order to generate training data, a plurality of medical image data can be input into the first sub-model to predict a plurality of corresponding focal spot shapes, and these focal spot shapes can then be input into the second sub-model to predict a plurality of corresponding focal spot degradation scores. The plurality of medical image data can then be combined with the plurality of corresponding focal spot degradation scores to form the training data for the composite model.

In other words, with the above sub-models, there is provided a dataset which links tube usage profiles (i.e., focal spot degradation) to focal spot shapes to position-dependent MTFs. In a specific example, this can thus be used to create training data for a supervised artificial intelligence (Al) model (i.e., the composite model). For example, the position-dependent MTFs can be used to create a dataset of either clinical or simulated patient scans that represent different levels of degradation. For each level of degradation, there can be provided a MTF profile. This can be employed via position-dependent convolution on reconstructed volumes of patient scans or simulated patient scans. This can therefore result in a patient database with resolutions (i.e., medical image data) degraded by different MTF profiles, corresponding to certain levels of focal spot degradation.

Continuing the example, the resulting patient database can then be used to train a CNN that will learn the mapping from medical image data (e.g., a clinical image) to a focal spot degradation level/score. As operations in the field can be performed on an infrequent basis, e.g., daily, many architectures can be considered, with computation time not necessarily being a bottleneck.

As an alternative to using a CNN for predicting the focal spot degradation level based on clinical scans, estimates of the state of degradation can be obtained by creating a composite model based on projection data or reconstructed images from routine calibration procedures - the repetitive nature of this data may ease the computational sophistication and/or increase the accuracy of degradation estimates.

Once deployed, for example, the composite model can output a focal spot degradation score, for example, a scalar value (0-100%), reflecting the percentage of degradation of the focal spot (and thus the X-ray tube). In this case, for example, 100% means that the tube is due for a replacement (i.e., is at full degradation). This can also generate valuable data from the field to monitor tube health and create further predictive models to take actions on the customer side (adapting protocols) and vendor side (scheduling timely replacements).

In some embodiments, step 230 can thus comprise generating training data using the first sub-model and the second sub-model; and training the composite model, using the generated training data, to predict, from medical image data, a focal spot degradation score.

In some embodiments, the method 200 can further comprise generating a third sub-model (in a step not shown) describing a representation of a relationship between a focal spot degradation score and an X-ray tube degradation score indicating a level of degradation in the X-ray tube which generated the focal spot. Generating/training the composite model (as in step 230) can then further be based on the third sub-model, such that the composite model can then be for predicting X-ray tube degradation. This can thus allow users to be provided with a more complete assessment of the medical imaging equipment's health, allowing a degradation of the X-ray tube to be predicted (based on the focal spot degradation, which can itself be derived from medical image data). In other words, by further basing the generating/training of the composite model on the third sub-model, the composite model can be configured to predict an X-ray tube degradation score (indicating a level of degradation in the X-ray tube which generated the focal spot) based on input medical image data, thus allowing users to be directly provided with a level of degradation in the X-ray tube, rather than just a level of degradation of the focal spot. In some embodiments, the third sub-model can comprise at least one of: a look-up table; a mathematical function; a regression model; a machine-learning model; and an algorithm. Of course, the skilled person would understand that this feature is not necessary and in other embodiments, for example, the focal spot degradation score can be predicted as a final output (and essentially used as a direct proxy for the degradation of the X-ray tube).

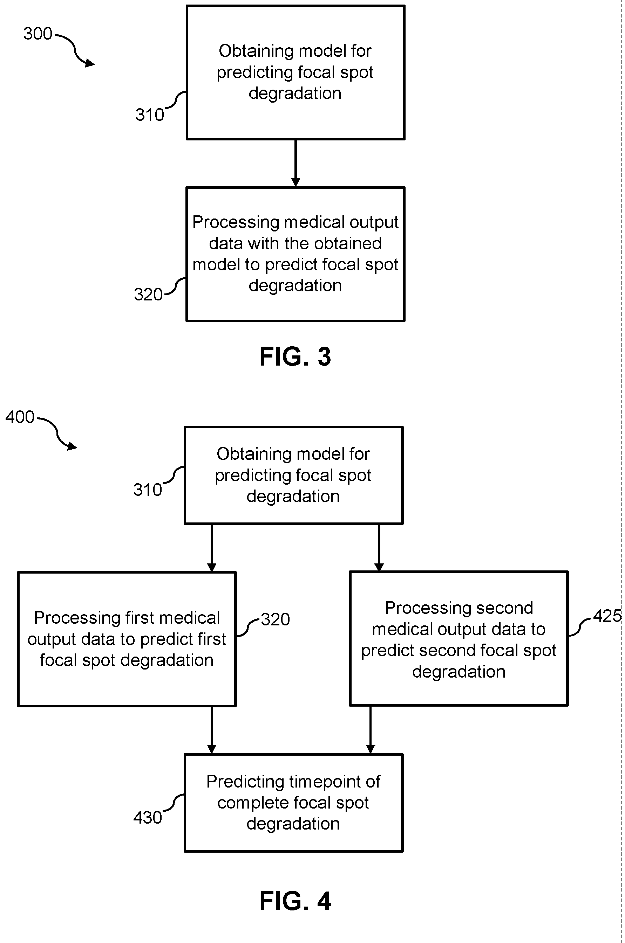

Referring now to Fig. 3, there is depicted a simplified flow diagram of a method 300 for predicting focal spot degradation according to a proposed embodiment.

Step 310 comprises obtaining a model for predicting focal spot degradation generated according to any of the herein-disclosed methods (e.g., according to method 100 or 200). For example, step 310 can comprise actually performing any herein-disclosed method for generating a model for predicting focal spot degradation, or for example, receiving the model from an external third party or from a memory storage or from a cloud-based service.

Step 320 comprises processing first medical image data from a single medical-imaging machine with the obtained model to predict a first focal spot degradation score. This allows a user to actually use the generated model in order to predict a focal spot degradation of an actual medical imaging system based on medical image data output by said medical imaging system. If the medical image data were from multiple medical-imaging machines (i.e., multiple CT systems or X-ray imaging systems) then their focal spots would not be the same and thus the focal spot degradation score would be incoherent.

Referring now to Fig. 4, there is depicted a flow diagram of a method 400 for predicting focal spot degradation according to a proposed embodiment. Steps 310 and 320 are substantially the same as have been described in relation to method 300 of Fig. 3.

Step 425 comprises processing second medical image data with the obtained model to predict a second focal spot degradation score, wherein the second medical image data was captured at a later point in time to the first medical image data and by the same medical imaging machine (e.g., by the same CT or X-ray imaging system).

Step 430 comprises predicting a timepoint when the medical imaging machine will reach complete focal spot degradation based on the first and second focal spot degradation scores and the times at which the first and second medical image data were captured. This thus facilitates the prediction of a timeline for complete focal spot degradation. This predicative capability can be especially useful for planning maintenance and replacement, ensuring continuous operation of medical imaging services without unexpected interruptions.

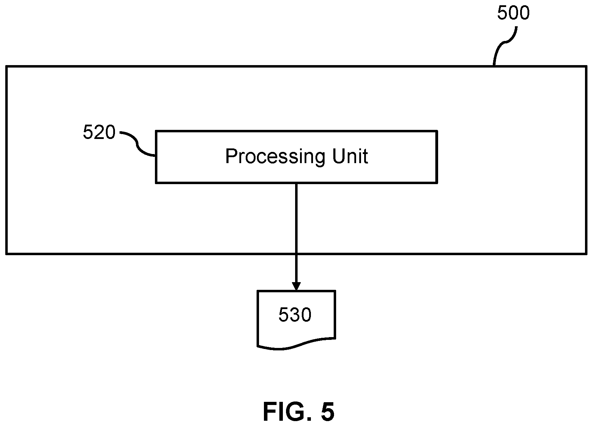

Referring now to Fig. 5, there is depicted a system 500 for generating a model for predicting focal spot degradation according to a proposed embodiment. The system 500 comprises a processing unit 520.

The processing unit 520 is configured to generate a first sub-model describing a representation of a relationship between medical image data and a focal spot shape; generate a second sub-model describing a representation of a relationship between a focal spot shape and a focal spot degradation score indicating a level of degradation in the focal spot's shape and/or size; and generate a composite model for predicting focal spot degradation based on the first sub-model and the second sub-model; wherein the medical image data comprises one of: CT projection data; a CT reconstructed image; X-ray projection data; and an X-ray reconstructed image. The system 500 thus generates an output 530 comprising the composite model for predicting focal spot degradation.

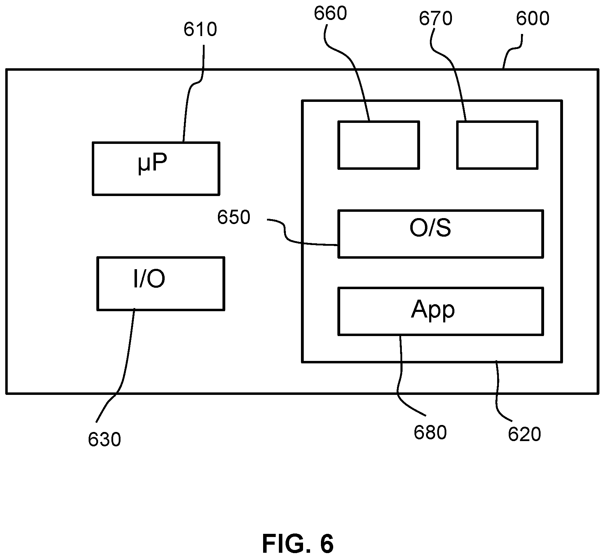

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g., connected via Internet). The computer 600 may include one or more processors 610, memory 620 and one or more I/O devices 630.

The processor 610 is a hardware device for executing software that can be stored in the memory 620. The memory 620 can have a distributed architecture, where various components are situated remote from one another, but can be accessed by the processor 610. The software in the memory 620 may include one or more separate programs.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to generating a model for predicting focal spot degradation. According to proposed concepts, a number of possible solutions may be implemented separately or jointly. That is, although these possible solutions may be described above separately, two or more of these possible solutions may be implemented in one combination or another.

## Claims

1. A computer-implemented method (100) of generating a model for predicting focal spot degradation, the method comprising:
generating a first sub-model (110) describing a representation of a relationship between medical image data and a focal spot shape;
generating a second sub-model (120) describing a representation of a relationship between a focal spot shape and a focal spot degradation score indicating a level of degradation in the focal spot's shape and/or size; and
generating a composite model (130) for predicting focal spot degradation based on the first sub-model and the second sub-model;
wherein the medical image data comprises one of: CT projection data; a CT reconstructed image; X-ray projection data; and an X-ray reconstructed image.

2. The computer-implemented method of claim 1, wherein the composite model is a machine-learning model, and wherein generating the composite model for predicting focal spot degradation comprises:
training the composite model (230), using the first and second sub-models, to predict, from medical image data, a focal spot degradation score.

3. The computer-implemented method of claim 2, wherein the composite model comprises at least one of: a convolutional neural network; and a transformer model.

4. The computer-implemented method of any of claims 1 to 3, wherein the first sub-model comprises a machine-learning model and generating the first sub-model comprises: training the first sub-model on training data (210) comprising simulated medical image data, wherein the simulated medical image data has been generated using different modulation transfer functions, MTFs, corresponding to different focal spot shapes.

5. The computer-implemented method of claim 4, wherein each modulation transfer function comprises a function of distance to an iso-center of the medical imaging machine.

6. The computer-implemented method of claim 5, wherein the distance to the iso-center comprises one of: tangential distance; and radial distance.

7. The computer-implemented method of any of claims 4 to 6, wherein the method further comprises: generating the different MTFs (205) using a simulated medical imaging machine with an adjustable focal spot shape.

8. The computer-implemented method of claims 1 to 7, wherein the second sub-model comprises at least one of: a look-up table; a mathematical function; a regression model; a machine-learning model; and an algorithm.

9. The computer-implemented method of any of claims 1 to 8, wherein the first sub-model comprises at least one of: a machine-learning model; an algorithm; a regression model; and a mathematical function.

10. The computer-implemented method of any of claims 1 to 9, wherein the method further comprises generating a third sub-model describing a representation of a relationship between a focal spot degradation score and an X-ray tube degradation score indicating a level of degradation in the X-ray tube which generated the focal spot; and generating the composite model is further based on the third sub-model, such that the composite model is for predicting X-ray tube degradation.

11. A computer-implemented method (300) for predicting focal spot degradation, the method comprising:
obtaining a model for predicting focal spot degradation (310) generated according to any of claims 1 to 10; and
processing first medical image data (320) from a single medical-imaging machine with the obtained model to predict a first focal spot degradation score.

12. The method of claim 11, wherein the method further comprises:
processing second medical image data (425) with the obtained model to predict a second focal spot degradation score, wherein the second medical image data was captured at a later point in time to the first medical image data and by the same medical imaging machine; and
predicting a timepoint when the medical imaging machine will reach complete focal spot degradation (430) based on the first and second focal spot degradation scores and the times at which the first and second medical image data were captured.

13. A computer program product comprising computer program code means which, when executed on a computing device having a processing system, cause the processing system to perform all of the steps of the computer-implemented method according to any of claims 1 to 12.

14. A system (500) for generating a model for predicting a level of focal spot degradation, the system comprising:
a processing unit (520) configured to:
generate a first sub-model describing a representation of a relationship between medical image data and a focal spot shape;
generate a second sub-model describing a representation of a relationship between a focal spot shape and a focal spot degradation score indicating a level of degradation in the focal spot's shape and/or size; and
generate a composite model for predicting focal spot degradation based on the first sub-model and the second sub-model;
wherein the medical image data comprises one of: CT projection data; a CT reconstructed image; X-ray projection data; and an X-ray reconstructed image.

15. The system of claim 14, wherein the composite model is a machine-learning model, and wherein generating the composite model for predicting focal spot degradation comprises training the composite model, using the first and second sub-models, to predict, from medical image data, a focal spot degradation score.
